# EUROPÄISCHE PATENTSCHRIFT

(11) **EP 3 666 235 B1**
(45) Veröffentlichungstag und Bekanntmachung des Hinweises auf die Patenterteilung: **08.12.2021**
(21) Anmeldenummer: 19214369.1
(22) Anmeldetag: 09.12.2019
(51) Int. Cl.: A61F 5/56

(54) **VORRICHTUNG ZUM VERHINDERN VON SCHNARCHEN UND APNOE**
DEVICE FOR PREVENTING SNORING AND APNEA
DISPOSITIF PERMETTANT D'EMPÊCHER LE RONFLEMENT ET L'APNÉE

(30) Priorität: 11.12.2018 DE 102018131715
(43) Veröffentlichungstag der Anmeldung: 17.06.2020
(73) Patentinhaber: Schmitt-Bylandt, Jürgen, 63571 Gelnhausen-Haitz (DE)
(72) Erfinder: Schmitt-Bylandt, Jürgen, 63571 Gelnhausen-Haitz (DE)
(74) Vertreter: Wolf, Michael

(56) Entgegenhaltungen:
- WO-A1-2016/169881
- DE-A1-102013 109 050
- DE-B3-102007 050 309

## Beschreibung

Die Erfindung betrifft eine Vorrichtung zum Verhindern von Schnarchen und Apnoe gemäß dem Oberbegriff des Patentanspruchs 1.

Eine Vorrichtung der eingangs genannten Art ist aus dem Patentdokument EP 2 131 801 B1 bekannt. Diese Vorrichtung besteht aus einem ersten, u-förmigen Schienenelement zur Oberkieferfassung und einem zweiten, u-förmigen Schienenelement zur Unterkieferfassung, wobei die Schienenelemente zum Bewirken eines Vorschubs des unterkieferseitigen, zweiten Schienenelements jeweils an ihren freien Schenkeln (also im Bereich der hinteren Backenzähne bzw. am abgewandten Ende zum Verbindungsbereich der beiden Schenkel) über ein eine federnde Verbindung zwischen den Schienenelementen schaffendes Verbindungselement miteinander verbunden ausgebildet sind.

DE 10 2013 109050 beschreibt eine Vorrichtung zum Verhindern von Schnarchen und Apnoe, umfassend ein erstes, u-förmiges Schienenelement zur Oberkieferfassung und ein zweites, u-förmiges Schienenelement zur Unterkieferfassung, wobei die Schienenelemente zum Bewirken eines Vorschubs des unterkieferseitigen, zweiten Schienenelements jeweils an ihren freien Schenkeln über eine federnde Verbindung zwischen den Schienenelementen schaffendes Verbindungselement miteinander verbunden ausgebildet sind. Der Erfindung liegt die Aufgabe zugrunde, eine Vorrichtung der eingangs genannten Art zu verbessern. Insbesondere soll auf effektive Weise die Haltbarkeit bzw. die Lebensdauer der bei manchen Benutzern stark belasteten Verbindungselemente erhöht werden.

Diese Aufgabe ist mit einer Vorrichtung der eingangs genannten Art durch die im Kennzeichen des Patentanspruchs 1 aufgeführten Merkmale gelöst.

Nach der Erfindung ist also vorgesehen, dass zur Entlastung des Verbindungselements ein die Schienenelemente miteinander verbindendes und ab Unterschreitung eines vorgegebenen Mindestvorschubs einer Bewegung des zweiten Schienenelements in Gegenvorschubrichtung entgegenwirkendes Halteelement vorgesehen ist.

Mit anderen Worten zeichnet sich die erfindungsgemäße Lösung somit dadurch aus, dass die Schienenelemente nicht mehr nur über das Verbindungselement bzw. die beiden Verbindungselemente, sondern zusätzlich auch noch über mindestens ein Halteelement (vorzugsweise zwei) miteinander verbunden ausgebildet sind. Dieses Halteelement ist dabei so an den Schienenelementen befestigt, dass es bei Unterschreitung eines vorgegebenen Mindestvorschubs einer weiteren Bewegung des zweiten Schienenelements in Gegenvorschubrichtung, also in Rückschubrichtung, entgegenwirkt. Das Halteelemente dient somit dazu, der unerwünschten Bewegung eine Kraft entgegen zu setzen, wobei der Betrag dieser Kraft von der Art des im Einzelnen benutzten Halteelements (hierauf wird weiter unten noch eingegangen) abhängt. Die erfindungsgemäße Maßgabe bewirkt somit, dass das Verbindungselement in Rückschubrichtung nur begrenzt belastet wird.

Andere vorteilhafte Weiterbildungen der erfindungsgemäßen Vorrichtung ergeben sich aus den abhängigen Patentansprüchen.

Der Vollständigkeit halber wird noch auf folgenden Stand der Technik hingewiesen:
Aus dem Dokument DE 20 2007 007 760 U1 ist eine Protrusionsschiene bekannt, bei der zum Verhindern von Schnarchen und Apnoe beidseitig zwischen der oberen und der unteren Zahnschniene ein als elastische Zug- und Druckfeder (siehe dort Absatz [0004]) ausgebildetes Verbindungselement vorgesehen ist. Verbindungselemente an den freien Enden der Zahnschienen sind bei dieser Lösung entsprechend nicht vorgesehen und auch nicht erforderlich.

Aus dem Dokument WO 2016/169881 A1 ist eine orthodontische Vorrichtung bekannt, bei der die beiden Enden des Verbindungselements (dort "biasing member" genannt) aber ebenfalls federnd miteinander verbunden ausgebildet sind.

Die erfindungsgemäße Vorrichtung einschließlich ihrer vorteilhaften Weiterbildungen gemäß der abhängigen Patentansprüche wird nachfolgend anhand der zeichnerischen Darstellung eines bevorzugten Ausführungsbeispiels näher erläutert.

Es zeigt schematisch
- Figur 1: in Seitenansicht die Vorrichtung zum Verhindern von Schnarchen und Apnoe mit dem erfindungsgemäßen Halteelement bei unbelasteten Schienenelementen;
- Figur 2: in Seitenansicht die Vorrichtung gemäß Figur 1 bei Unterschreitung eines vorgegebenen Mindestvorschubs und bei entsprechend gespanntem Halteelement;
- Figur 3: in Draufsicht die Vorrichtung gemäß Figur 1;
- Figur 4: in Unteransicht die Vorrichtung gemäß Figur 1;
- Figur 5: in Vorderansicht die Vorrichtung gemäß Figur 1; und
- Figur 6: in Rückansicht die Vorrichtung gemäß Figur 1.

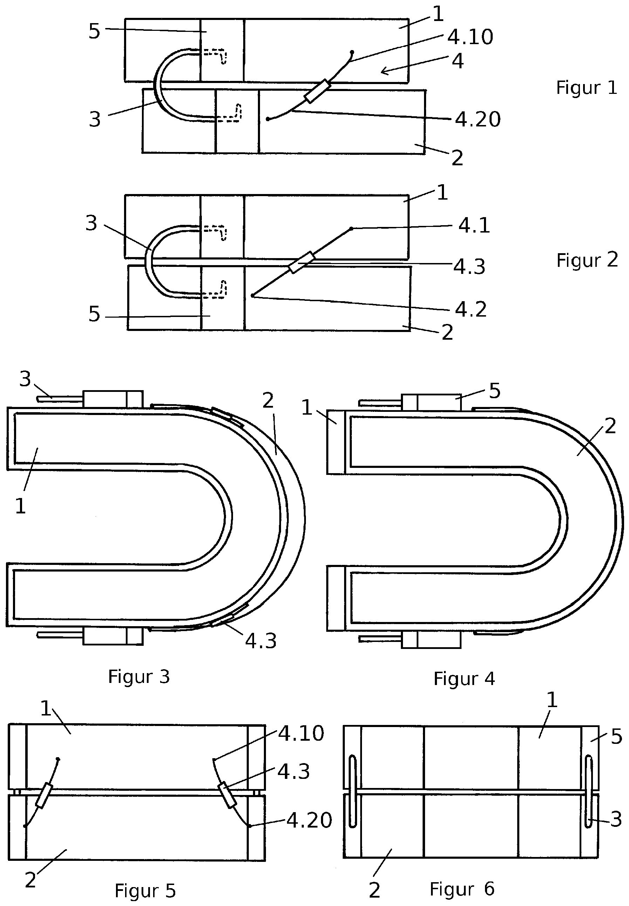

Die in den Figuren 1 bis 6 dargestellte Vorrichtung zum Verhindern von Schnarchen und Apnoe besteht zunächst in bekannter Weise aus einem ersten, u-förmigen Schienenelement 1 zur Oberkieferfassung und einem zweiten, u-förmigen Schienenelement 2 zur Unterkieferfassung, wobei die Schienenelemente 1, 2, die vorzugsweise aus Kunststoff gebildet sind, zum Bewirken eines Vorschubs des unterkieferseitigen, zweiten Schienenelements 2 jeweils an ihren freien Schenkeln über ein eine federnde Verbindung zwischen den Schienenelementen 1, 2 schaffendes Verbindungselement 3 miteinander verbunden ausgebildet sind. Dabei ist besonders bevorzugt vorgesehen, dass die Verbindungselemente 3 jeweils an Enden der Schienenelemente 1, 2 angeordnet sind, und zwar vorzugsweise solchen Enden, die einem zur Aufnahme von Schneidezähnen dienenden Abschnitt der Schienenelemente 1, 2 gegenüberliegend angeordnet sind.

Weiterhin ist bevorzugt vorgesehen (nicht extra dargestellt), dass die Schienenelemente 1, 2 (nach Durchführung eines entsprechenden Abdrucks) an Zahnreihen eines Benutzers formangepasst ausgebildet sind.

Bezüglich des Verbindungselements 3, das, wie insbesondere aus den Figuren 3, 4 und 6 ersichtlich, zweifach vorhanden und jeweils an den freien Schenkeln der Schienenelemente 1, 2 angeordnet ist, ist bevorzugt vorgesehen, dass dieses aus Metall, insbesondere aus einer Formgedächtnislegierung (siehe https:// de.wikipedia.org/w/index.php?title=Formged%C3%A4chtnislegierung&oldid=176600432), gebildet ist. Ferner ist, wie in den Figuren dargestellt, bevorzugt vorgesehen, dass das Verbindungselement 3 drahtförmig ausgebildet ist.

Zur Befestigung der Verbindungselemente 3 an den Schienenelementen 1, 2 ist darüber hinaus vorzugsweise vorgesehen, dass das Verbindungselement 3 beidseitig über jeweils einen Aufnahmekörper 5 mit jeweils einem Schienenelement 1, 2 verbunden ausgebildet ist. Dieser Aufnahmekörper 5 besteht dabei vorzugsweise aus dem gleichen Material wie die Schienenelemente 1, 2 selbst.

Wesentlich für die erfindungsgemäße Vorrichtung ist nun, dass zur Entlastung des Verbindungselements 3 ein die Schienenelemente 1, 2 miteinander verbindendes und ab Unterschreitung eines vorgegebenen Mindestvorschubs einer Bewegung des zweiten Schienenelements 2 in Gegenvorschubrichtung entgegenwirkendes Halteelement 4 vorgesehen ist bzw. dass die Vorrichtung zur Entlastung des Verbindungselements 3 ein die Schienenelemente 1, 2 miteinander verbindendes und ab Unterschreitung eines vorgegebenen Mindestvorschubs einer Bewegung des zweiten Schienenelements 2 in Gegenvorschubrichtung entgegenwirkendes Halteelement 4 aufweist bzw. umfasst.

Um eine zulässige Maximalbelastung des Verbindungselements 3 von Anfang an genau festzulegen, ist dabei mit Verweis auf Figur 2 vorgesehen, dass das Halteelement 4 ab Unterschreitung eines vorgegebenen Mindestvorschubs eine Bewegung des zweiten Schienenelements 2 in Gegenvorschubrichtung unterbindend ausgebildet ist. Bei der dargestellten Lösung ist dabei dadurch, dass das als Stahlseil ausgebildete Halteelemente 4 letztlich durch die Muskulatur des Benutzers höchstens nur ganz geringfügig gedehnt werden kann, gewährleistet, dass eine weitere Verschiebung des unteren Schienenelements 2 nach hinten (also nach links in Figur 2) ausgeschlossen ist.

Wie bereits zum Verbindungselement 3 erläutert, ist dabei auch das Halteelement 4 vorzugsweise zweifach vorgesehen, nämlich an beiden Seiten der Schienenelemente 1, 2.

Wie aus den Figuren ersichtlich, ist ferner bevorzugt vorgesehen, dass eine Befestigungsstelle 4.1 des Halteelements 4 am ersten Schienenelement 1 weiter entfernt vom Verbindungselement 3 als eine Befestigungsstelle 4.2 des Halteelements 4 am zweiten Schienenelement 2 angeordnet ist. Noch etwas genauer betrachtet, ist dabei besonders bevorzugt zwischen den freien, für die Backenzähne vorgesehenen Schenkeln des Schienenelements 1, 2 ein für die Schneidzähne vorgesehener Übergangsbereich (also am Fuß des "U"s) vorgesehen, wobei dann darüber hinaus das Halteelement 4 an den freien Schenkeln einerseits übergangsbereichsseitig am ersten Schienenelement 1 und andererseits verbindungselementseitig am zweiten Schienenelement 2 befestigt ist. Das Halteelement 4 verläuft somit im Grunde ausgehend von den Schneidezähnen des Benutzers von vorne oben nach hinten unten, wobei die Gesamtanordnung des Halteelements 4 insgesamt vorzugsweise so nah wie möglich am Verbindungselement 3 erfolgt, damit das Öffnen der Vorrichtung bzw. des Mundes des Benutzers so wenig wie möglich behindert wird.

Bezüglich des Halteelements 4 selbst ist dabei, wie oben bereits angedeutet, besonders bevorzugt vorgesehen, dass dieses ausschließlich zur Übertragung von Zugkräften ausgebildet ist. Hierzu ist das Halteelement 4 besonders bevorzugt zumindest abschnittsweise seilartig ausgebildet.

Ferner ist besonders bevorzugt vorgesehen, dass das Halteelement 4 aus zwei miteinander verbundenen Teilstücken 4.10, 4.20 gebildet ist. Dabei ist weiterhin zur Verbindung der beiden Teilstücke 4.10, 4.20 besonders bevorzugt ein Hülsenelement 4.3 vorgesehen. Dieses ist dabei vorzugsweise aus Metall, besonders bevorzugt aus einer Formgedächtnislegierung, gebildet. Außerdem ist dabei bevorzugt vorgesehen, dass ein Ende des einen und ein Ende des anderen Teilstücks 4.10, 4.20 parallel nebeneinander liegend vom Hülsenelement 4.3 umschlossen ausgebildet sind. Auf diese Weise lässt sich eine sehr einfache Verstellbarkeit der Länge des Halteelements 4 erreichen.

### Bezugszeichenliste

- 1: erstes Schienenelement zur Oberkieferfassung
- 2: zweites Schienenelement zur Unterkieferfassung
- 3: Verbindungselement
- 4: Halteelement
- 4.1: Befestigungsstelle
- 4.2: Befestigungsstelle
- 4.3: Hülsenelement
- 4.10: Teilstück
- 4.20: Teilstück
- 5: Aufnahmekörper

## Patentansprüche

1. Vorrichtung zum Verhindern von Schnarchen und Apnoe, umfassend ein erstes, u-förmiges Schienenelement (1) zur Oberkieferfassung und ein zweites, u-förmiges Schienenelement (2) zur Unterkieferfassung, wobei die Schienenelemente (1, 2) zum Bewirken eines Vorschubs des unterkieferseitigen, zweiten Schienenelements (2) jeweils an ihren freien Schenkeln über ein eine federnde Verbindung zwischen den Schienenelementen (1, 2) schaffendes Verbindungselement (3) miteinander verbunden ausgebildet sind,
**dadurch gekennzeichnet,**
**dass** zur Entlastung des Verbindungselements (3) ein die Schienenelemente (1, 2) miteinander verbindendes und ab Unterschreitung eines vorgegebenen Mindestvorschubs einer Bewegung des zweiten Schienenelements (2) in Gegenvorschubrichtung entgegenwirkendes Halteelement (4) vorgesehen ist.

2. Vorrichtung nach Anspruch 1,
**dadurch gekennzeichnet,**
**dass** das Halteelement (4) ab Unterschreitung eines vorgegebenen Mindestvorschubs eine Bewegung des zweiten Schienenelements (2) in Gegenvorschubrichtung unterbindend ausgebildet ist.

3. Vorrichtung nach Anspruch 1 oder 2,
**dadurch gekennzeichnet,**
**dass** eine Befestigungsstelle (4.1) des Halteelements (4) am ersten Schienenelement (1) weiter entfernt vom Verbindungselement (3) als eine Befestigungsstelle (4.2) des Halteelements (4) am zweiten Schienenelement (2) angeordnet ist.

4. Vorrichtung nach einem der Ansprüche 1 bis 3, wobei zwischen den freien, für die Backenzähne vorgesehenen Schenkeln des Schienenelements (1, 2) ein für die Schneidzähne vorgesehener Übergangsbereich vorgesehen ist,
**dadurch gekennzeichnet,**
**dass** das Halteelement (4) an den freien Schenkeln einerseits übergangsbereichsseitig am ersten Schienenelement (1) und andererseits verbindungselementseitig am zweiten Schienenelement (2) befestigt ist.

5. Vorrichtung nach einem der Ansprüche 1 bis 4,
**dadurch gekennzeichnet,**
**dass** das Halteelement (4) ausschließlich zur Übertragung von Zugkräften ausgebildet ist.

6. Vorrichtung nach einem der Ansprüche 1 bis 5,
**dadurch gekennzeichnet,**
**dass** das Halteelement (4) zumindest abschnittsweise seilartig ausgebildet ist.

7. Vorrichtung nach einem der Ansprüche 1 bis 6,
**dadurch gekennzeichnet,**
**dass** das Halteelement (4) aus zwei miteinander verbundenen Teilstücken (4.10, 4.20) gebildet ist.

8. Vorrichtung nach Anspruch 7,
**dadurch gekennzeichnet,**
**dass** zur Verbindung der Teilstücke (4.10, 4.20) ein Hülsenelement (4.3) vorgesehen ist.

9. Vorrichtung nach Anspruch 8,
**dadurch gekennzeichnet,**
**dass** das Hülsenelement (4.3) aus Metall gebildet ist.

10. Vorrichtung nach Anspruch
**dadurch gekennzeichnet,**
**dass** ein Ende des einen und ein Ende des anderen Teilstücks (4.10, 4.20) parallel nebeneinander liegend vom Hülsenelement (4.3) umschlossen ausgebildet sind.

## Claims

1. A device for preventing snoring and apnea, comprising a first U-shaped splint element (1) for holding the upper jaw and a second U-shaped splint element (2) for holding the lower jaw, wherein the splint elements (1, 2) are formed to be connected to one another in each case at their free legs via a connecting element (3) creating a resilient connection between the splint elements (1, 2) in order to effect an advancement of the second splint element (2) on the lower jaw side,
**characterized in**
**that**, in order to take load off the connecting element (3), a holding element (4) is provided which connects the splint elements (1, 2) to one another and counteracts a movement of the second splint element (2) in the opposite advancement direction upon falling below a predetermined minimum advancement.

2. The device according to claim 1,
**characterized in**
**that** the holding element (4) is formed to prevent a movement of the second splint element (2) in the opposite advancement direction upon falling below a predetermined minimum advancement.

3. The device according to claim 1 or 2,
**characterized in**
**that** an attachment point (4.1) of the holding element (4) on the first splint element (1) is arranged farther away from the connecting element (3) than an attachment point (4.2) of the holding element (4) on the second splint element (2).

4. The device according to any one of claims 1 to 3, wherein a transition region provided for the incisors is provided between the free legs of the splint element (1, 2) provided for the molars,
**characterized in**
**that** the holding element (4) is attached, on the one hand, at the free legs to the first splint element (1) on the transition region side and, on the other hand, to the second splint element (2) on the connecting element side.

5. The device according to any one of claims 1 to 4,
**characterized in**
**that** the holding element (4) is formed exclusively for transmitting tensile forces.

6. The device according to any one of claims 1 to 5,
**characterized in**
**that** the holding element (4) is formed, at least in sections, in a rope-like manner.

7. The device according to any one of claims 1 to 6,
**characterized in that**
that the holding element (4) is formed from two interconnected sections (4.10, 4.20).

8. The device according to claim 7,
**characterized in**
**that** a sleeve element (4.3) is provided for connecting the sections (4.10, 4.20).

9. The device according to claim 8,
**characterized in that**
that the sleeve element (4.3) is formed from metal.

10. The device according to claim 8,
**characterized in**
**that** one end of the one section and one end of the other section (4.10, 4.20) are formed to be parallel adjacent to each other and enclosed by the sleeve element (4.3).

## Revendications

1. Dispositif anti-ronflements et anti-apnée, comprenant un premier élément de rail (1) en forme de U, destiné à saisir la mâchoire supérieure et un deuxième élément de rail (2) en forme de U, destiné à saisir la mâchoire inférieure, sachant que pour provoquer une avance du deuxième élément de rail (2) côté mâchoire inférieure, les éléments de rail (1, 2) étant conçus en étant reliés l'un à l'autre respectivement sur leurs branches libres par l'intermédiaire d'un élément de liaison (3) créant une liaison résiliente entre les éléments de rail (1, 2),
**caractérisé en ce que**
pour décharger l'élément de liaison (3), il est prévu un élément de retenue (4) reliant l'un à l'autre les éléments de rail (1, 2) et s'opposant dans la direction anti-avance à un déplacement du deuxième élément de rail (2), à partir d'une non-atteinte d'une avance minimale prédéfinie.

2. Dispositif selon la revendication 1,
**caractérisé en ce que**
l'élément de retenue (4) est conçu pour éviter un déplacement du deuxième élément de rail (2) dans la direction anti-avance à partir d'une non-atteinte d'une avance minimale prédéfinie.

3. Dispositif selon la revendication 1 ou 2,
**caractérisé en ce qu'**
un point de fixation (4.1) de l'élément de retenue (4) sur le premier élément de rail (1) est placé en étant plus éloigné de l'élément de liaison (3) qu'un point de fixation (4.2) de l'élément de retenue (4) sur le deuxième élément de rail (2).

4. Dispositif selon l'une quelconque des revendications 1 à 3, sachant qu'entre les branches libres de l'élément de rail (1, 2), prévues pour les molaires étant prévue une zone de transition prévue pour les incisives,
**caractérisé en ce que**
l'élément de retenue (4) est fixé sur les branches libres, d'une part sur le côté zone de transition sur le premier élément de rail (1) et d'autre part sur le côté élément de liaison sur le deuxième élément de rail (2).

5. Dispositif selon l'une quelconque des revendications 1 à 4,
**caractérisé en ce que**
l'élément de retenue (4) est conçu exclusivement pour transmettre des forces de traction.

6. Dispositif selon l'une quelconque des revendications 1 à 5,
**caractérisé en ce que**
l'élément de retenue (4) est conçu au moins par portions à la manière d'un câble.

7. Dispositif selon l'une quelconque des revendications 1 à 6,
**caractérisé en ce que**
l'élément de retenue (4) est conçu en deux pièces partielles (4.10, 4.20) reliées l'une à l'autre.

8. Dispositif selon la revendication 7,
**caractérisé en ce que**
pour relier les pièces partielles (4.10, 4.20), il est prévu un élément en douille (4.3).

9. Dispositif selon la revendication 8,
**caractérisé en ce que**
l'élément en douille (4.3) est conçu en métal.

10. Dispositif selon la revendication 8,
**caractérisé en ce qu'**
une extrémité de l'une et une extrémité de l'autre pièce partielle (4.10, 4.20) sont conçues placées côte à côte à la parallèle, en étant entourées par l'élément en douille (4.3).
